# EUROPEAN PATENT SPECIFICATION

(11) **EP 2 155 246 B1**
(45) Date of publication and mention of the grant of the patent: **13.03.2013**
(21) Application number: 07748678.5
(22) Date of filing: 11.05.2007
(51) Int. Cl.: C12N 7/00

(54) **PRODUCTION OF A HOMOGENEOUS CELL LINE HIGHLY PERMISSIVE TO PORCINE CIRCOVIRUS TYPE 2 (PCV2) INFECTION**
HERSTELLUNG EINER HOMOGENEN ZELLLINIE, DIE HOCHEMPFÄNGLICH FÜR EINE INFEKTION MIT DEM SCHWEINE-CIRCOVIRUS TYP 2 (PCV2) IST
PRODUCTION D'UNE LIGNÉE DE CELLULES HOMOGÈNES HAUTEMENT SENSIBLES À UNE INFECTION PAR LE CIRCOVIRUS PORCIN DE TYPE 2 (PCV2)

(43) Date of publication of application: 24.02.2010
(73) Proprietor: Temasek Life Sciences Laboratory Limited, Singapore 117604 (SG)
(72) Inventor: LAU, Hui Ling, Adeline, 117604 Singapore (SG); LAU, Siew Kee, Jennifer, 140090 Singapore (SG); KWANG, Hwei-Sing, 117604 Singapore (SG)
(74) Representative: Brearley, Helen Rebecca
(86) International application number: PCT/SG2007/000133
(87) International publication number: WO 2008/140414

(56) References cited:
- WO-A2-2006/113435
- WO-A2-2006/132605
- AU-A- 9 355 598
- RU-C2- 2 201 960
- KIM H S ET AL: "ENHANCED REPLICATION OF PORCINE REPRODUCTIVE AND RESPIRATORY SYNDROME (PRRS) VIRUS IN A HOMOGENEOUS SUBPOPULATION OF MA-104 CELL LINE" ARCHIVES OF VIROLOGY, SPRINGER WIEN, AT LNKD- DOI:10.1007/BF01313785, vol. 133, 1 January 1993 (1993-01-01), pages 477-483, XP002027529 ISSN: 0304-8608
- ZHU ET AL: "Enhanced replication of porcine circovirus type 2 (PCV2) in a homogeneous subpopulation of PK15 cell line" VIROLOGY, ACADEMIC PRESS,ORLANDO, US LNKD- DOI:10.1016/J.VIROL.2007.08.014, vol. 369, no. 2, 14 November 2007 (2007-11-14), pages 423-430, XP022344146 ISSN: 0042-6822
- CHEUNG A.K. AND BOLIN S.R.: 'Kinetics of porcine circovirus type 2 replication' ARCHIVES OF VIROLOGY vol. 147, no. 1, 2002, pages 43 - 58, XP001157507
- MATEUSEN B. ET AL.: 'Susceptibility of pig embryos to porcine circovirus type 2 infection' THERIOGENOLOGY vol. 61, no. 1, January 2004, pages 91 - 101, XP008124765
- HIRAI T. ET AL.: 'Infectivity of porcine cicovirus 1 and circovirus 2 in primary porcine hepatocyte and kidney cell cultures' THE JOURNAL OF VETERINARY MEDICAL SCIENCE/THE JAPANESE SOCIETY OF VETERINARY SCIENCE vol. 68, no. 2, February 2006, pages 179 - 182, XP008124832
- CHEUNG A.K.: 'Transcriptional Analysis of Porcine Circovirus Type 2' VIROLOGY vol. 305, no. 1, 2003, pages 168 - 180, XP008124766
- MEEHAN B.M. ET AL.: 'Characterization of novel circovirus DNAs associated with wasting syndromes in pigs' THE JOURNAL OF GENERAL VIROLOGY vol. 79, no. PART 9, September 1998, pages 2171 - 2179, XP002090386
- 'Passage number effects in cell line', [Online] ATCC Retrieved from the Internet: <URL:http://www.genengnews.com/transfection /ATCC_TechBulletin_7_Final_06_07.pdf>
- TISCHER I ET AL: "REPLICATION OF PORCINE CIRCOVIRUS: INDUCTION BY GLUCOSAMINE AND CELL CYCLE DEPENDENCE", ARCHIVES OF VIROLOGY, SPRINGER WIEN, AT, vol. 96, no. 1/02, 1 January 1987 (1987-01-01), pages 39-57, XP008017306, ISSN: 0304-8608, DOI: DOI:10.1007/BF01310989
- DEZENGRINI ET AL: "Selection and characterization of canine, swine and rabbit cell lines resistant to bovine viral diarrhea virus", JOURNAL OF VIROLOGICAL METHODS, ELSEVIER BV, NL, vol. 137, no. 1, 1 October 2006 (2006-10-01), pages 51-57, XP005600250, ISSN: 0166-0934, DOI: DOI:10.1016/J.JVIROMET.2006.05.032

## Description

### Background of the Invention

The present invention relates to the production of porcine circovirus type 2 (PCV2). More particularly, the invention relates to continuous cell lines that are highly susceptible to infection with PCV2 and to methods for the production of PCV2 using the cell lines.

Porcine circovirus (PCV) is a small, non-enveloped, circular, single-stranded DNA virus classified in the Circoviridae family. Murphy, F A., Fauquet, C M., Bishop, D H L., Ghabrial, S A., Jarvis, A W., Martelli, G P.; Mayo, M A., Summers, M D. Virus taxonomy. Sixth report of the International Committee on Taxonomy of Viruses. New York, N.Y: Springer-Verlag; 1995. pp. 166-168. It was originally identified and described as a contaminant of a porcine kidney cell line. Tischer, I., Gelderblom, H., Vettermann, W., Koch, M. A. A very small porcine virus with circular single-stranded DNA. Nature. 1982:295:64-66. Recently, PCV has been associated with a disease of pigs, the post-weaning multi-systemic wasting syndrome (PMWS), first observed in Western Canada. Ellis, J., Hassard, L., Clark, E., Harding, J., Allan, G., Willson, P., Strokappe, J., Martin, K., McNeilly, F., Meehan, F., Todd, D., Haines, D. Isolation of circovirus from lesions ofpigs with postweaning multisystemic wasting syndrome. Can. Vet. J., 1998; 39:44-51; Harding, J. C. S., Clark, E. G. Recognizing and diagnosing postweaning multisystemic wasting syndrome (PMWS). Swine Health Prod. 1997; 5:201-203; Jue Liu, Isabelle Chen, and Jimmy Kwang, J. Virol. 2005: 79(13); 8262-74. PWMS has emerged as a major disease that poses a significant threat to the economics of the global swine industry. After its first appearance in Canada, PMWS has now spread to the United States, Europe and Asia. The syndrome mainly affects pigs between 6 and 14 weeks of age. It tends to be slow and progressive with a high fatality rate in affected pigs.
*See* *http:*//*www.aphis.usda.gov*/*vs*/*ceah*/*cei*/*taf*/*emergingdiseasenotice_files*/*pmws 0301.htm.*

The clinical signs of PMWS are quite variable. Affected pigs may show signs of chronic wasting, respiratory distress, diarrhea, incoordination, paralysis, pale skin color and blue ears. Pigs usually demonstrate a decrease in growth rate and, occasionally, jaundice.

The diagnosis of PMWS is based on the age of affected pigs, typical wasting appearance and necropsy lesions. Microscopic and imunohistochemical examination of tissues reveals unique lung and lymphoid tissue lesions with the presence of PCV2. *Id.*

Antibacterial medication is usually ineffective in treating PWMS and currently no vaccines are available. Prevention of the syndrome is based on biosecurity precautions and good husbandry practices.

PCV2 has also been found in association with other diseases including porcine dermatitis and nephropathy syndrome ("PDNS"), congenital tremors (CT-All) reproductive disorders, prenatal myocarditis and proliferative and necrotizing pneumonia.

Vaccines employing PCV2 antigens have shown some initial success in preventing the PMWS. Fenaux, M., et al., A chimeric porcine circovirus (PCV) with the immunogenic capsid gene of the pathogenic PCV type 2 (PCV2) cloned into the genomic backbone of the nonpathogenic PCV1 induces protective immunity against PCV2 infection in pigs. J. Virol., 2004. 78(12): p. 6297-303; Blanchard, P. et al., Protection contre la maladie d'amaigrissement du porcelet (MAP) par vaccines a ADNet proteines recombinantes. Journees de la Recherche Porcine en France, 2004. 36: p. 345-352; Blanchard, P., et al., Protection of swine against porcine multisystemic wasting syndrome (PMWS) by porcine circovirus type 2 (PCV2) proteins. Vaccine, 2003. 21: p. 4565-4575; Pogranichniy, R. et al. Efficacy of inactivated PCV2 vaccines for preventing PMWS in CDCD pigs. American Association of Swine Veterinarians. 2004. Des Moines, Iowa. However, an effective vaccine is not currently available.

The development of vaccines, diagnostic agents and therapies for PMWS and other diseases associated with PCV2 viral infections will require efficient and reliable means for producing the virus in substantial quantities. PCV2 virus stocks have conventionally been produced by culturing the virus in porcine kidney cell-line PK15. The virus titers yielded from PK15 cell cultures, expressed as 50% tissue culture infectious dosage ("TCID₅₀") per milliliter, usually ranged from 10⁴-10⁵ and could never exceed 10⁵. Immunofluorescence stainings of infected PK15 cell cultures have revealed that only about 40% of the cell population is susceptible to the PCV2 infection.

A need exists for a continuous cell line that is highly permissive to PCV2 infection and that reliably produces virus in high titers over extended periods of time.

### Brief Description of the Drawings

Figure 1 shows immunofluorescence assay results demonstrating percentage infection on uncloned and cloned PK15 cell monolayers infected with PCV2 on 3 days post-infection. A, B, C and D represent mock-infected PK15 monolayer (negative control), infected PK15 monolayer, low-permissive and high-permissive (clone C1) subcloned monolayers respectively.
Figure 2 shows PCV2 attachment onto surface membrane of PK15 cell line, low- and high-permissive cell clones after 1 hour adsorption at 37°C.
Figure 3 shows growth curves of PK15 and clone C1 cell populations over 48 hours.
Figure 4 shows PCV2 virus yields generated in parental PK15 and cloned C1 cell lines over 4 passages.
Figure 5 shows PCV2 virus genome synthesis in parental PK15 and cloned C1 cell lines.

### Summary of the Invention

The present invention provides a continuous cell line that is highly permissive to PCV2 infection. In another embodiment, the invention provides a method for producing a substantially homogeneous cell line that is highly permissive to PCV2 infection, which comprises (1) cultivating a heterogeneous cell population that contains cells of varying susceptibility to PCV2 infection; (2) diluting the cell culture and placing aliquots of the diluted cells into separate vessels such that each vessel contains about one cell; (3) adding PCV2 to each vessel; (4) culturing the cells and identifying a vessel that contains cells that are susceptible to PCV2 infection; and (5) culturing and maintaining a cell line from such cells wherein said heterogenous cell population is a PK15 cell population. In a particular embodiment, the invention provides a continuous cell line designated PK15-C1. In yet another embodiment, the invention provides a method for the continuous production of porcine circovirus type 2 ("PCV2"), which comprises infecting a porcine kidney cell line PK15-C1 with said virus, growing said cell line under conditions suitable for cell growth; and recovering said virus produced by said cell line, wherein said porcine kidney cell line PK15-C1 is the cell line PK15-C1 deposited with the American Type Culture Collection on March 20, 2007 as PTA-8244.

### Detailed Description of the Invention

In accordance with the invention, it has been discovered that the population of cells in the PK15 porcine kidney cell line is heterogeneous with respect to permissivity to the PCV2 infection. The cell line has been found to contain cells of both low- and high-permissivity to viral infection. The relatively low virus titers produced by PCV2-infected PK15 are attributable to the heterogeneity of the cell line.

Homogeneous cell lines of this invention may be produced by cloning single cells and identifying resulting cultures that are highly susceptible to PCV2 infection. While the PKI5 cell line is a preferred cell line for use in the methods of this invention, other cell lines that are susceptible to infection with PCV2 may also be used to produce homogeneous virus-producing cell lines.

A culture of a heterogeneous cell population is first diluted into aliquots containing single cells. The single-cell aliquots are placed into individual vessels, such as the wells of a microtiter plate, and are suspended in a nutrient medium that contains nutrients, growth factors and buffers necessary for replication of the cells. They are then incubated under thermal and atmospheric conditions conducive to cell growth. Following cell growth, e.g., to a continuous monolayer, an infectious amount of virus is added to each aliquot and the cells are cultured until a suitable phase of virus production is achieved.

Virus titers of each aliquot are determined, e.g., by immunofluorescence assay according to the following protocol: Infected cells are fixed with 4% paraformaldehyde for 15 minutes at 72 hours post-infection (pi), and incubated with PCV2 ORF-I antibody followed by anti-guinea pig fluorescein isothyocianate (FITC), each for 1 hr at 37°C with washing of cells one time with phosphate buffered saline between each step. The staining results are observed under an Olympus fluorescent microscope and cells are scored for their ability to produce high virus titers. High virus-producing cell lines are then advantageously re-cloned and highly permissive cells are selected.

A preferred cell line produced in accordance with the invention was derived from cell line PK15 (ATCC CCL-33) and has been designated clone C1. It is referred to herein as PK15-C1. The results presented herein show that clonal C1 cell population is more susceptible to PCV2 infection than are PK15 cells. Therefore, PK15-C1 is a more effective cell line for the production of high PCV2 virus yield than the parental PK15 cell line.

PK15-C1 has been deposited with the American Type Culture Collection with accession number PTA-8244.

The invention is further illustrated by the following examples, which are not intended to limit the invention.

### Example 1

### Production of PK15-C1

The PK15 parent cell line was maintained in Eagle's minimum essential medium (MEM), supplemented with 5% fetal bovine serum (FBS), 2.2 g/L sodium bicarbonate, 2mM L-glutamine, 1.0 mM sodium pyruvate and antibiotics. Cloning of PK15 cells was performed by the limiting-dilution method. The cells were trypsinized, diluted at a mean concentration of 1 cell/well in MEM /20% FBS /60% conditioned media and dispensed into 96-well tissue culture plates. The wells were immediately screened for single cells and marked, and the plates were incubated at 37°C in an atmosphere of 5% CO₂. Following the identification of cell monolayers from the initial cloning, these subclones were subjected to another round of further cloning.

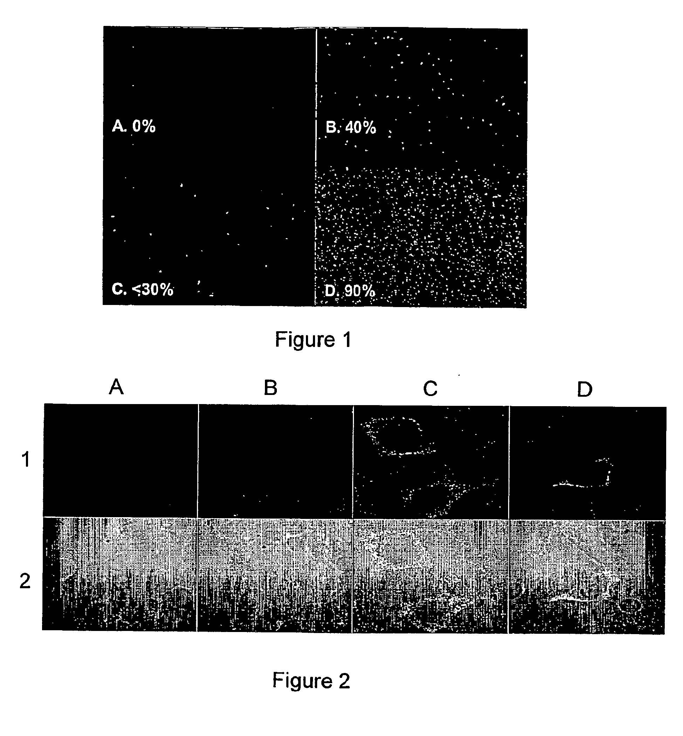

The PK15 parent and cloned cell populations were screened by immunofluorescence assay (IFA) for high- and low-permissive cells. The cells were seeded to 70% confluency in 96-well plates and infected with PCV2 with a titer of about 10⁵ TCID₅₀/ml at 6 hours post-seeding. Glucosamine (300mM) was added to the infected cells within 24 hours of infection, and the cells were maintained in MEM/5% FBS at 37°C/5% CO₂. Positive and negative controls used in this experiment were PCV2 virus supernatant (s/n) of 10⁵ TCID₅₀/ml and MEM/5% FBS respectively. The cells were fixed with 4% paraformaldehyde, and IFA was performed at 72 hours post-infection (pi). IFA results demonstrated highest of 90% PCV2 infection in high-permissive clone C1 cells, compared to only 40% PCV2 infection in PK15 parent cells, and less than 20% infection in the remaining low-permissive cell clones (Figure 1). In addition, a virus attachment assay was carried out to observe the affinity of PCV2 to the cell surface membrane of each cell clone. Each cell clone was seeded onto chamber slides and incubated overnight as above to obtain 70% confluency. PCV2 of 10⁷ TCID₅₀/ml was added to the cells for 1 hour adsorption at 37°C and fixed with 4% paraformaldehyde. IFA was performed as above, however using PCV2 ORF-2 antibody for the primary antibody. After the final washing, the stained cells were mounted with fluorescent mounting media and observed under a Zeiss Meta inverted confocal microscope. Confocal microscopy results demonstrating PCV2 attachment onto cell surface membranes of cloned and uncloned PK15 cells after 1 hour adsorption at 37° C are shown in Figure 2. A, B, C and D represent mock-infected PK15 monolayer (negative control), infected low-permissive, high-permissive (clone C1) subcloned monolayers and infected PK15 monolayer respectively. 1) FITC fluorescent staining image. 2) Overlay of light phase and green fluorescence image. The spread and intensity of fluorescence, indicating PCV2 attachment to the cell surface membrane, was observed most intensely on high-permissive cell clone C1, followed by uncloned PK15 cells and low-permissive cell clones. These results suggested that clone C1 is most susceptible to PCV2 infection, and therefore selected for propagation of PCV2 virus.

### Example 2

### Characterization of PK15-C1

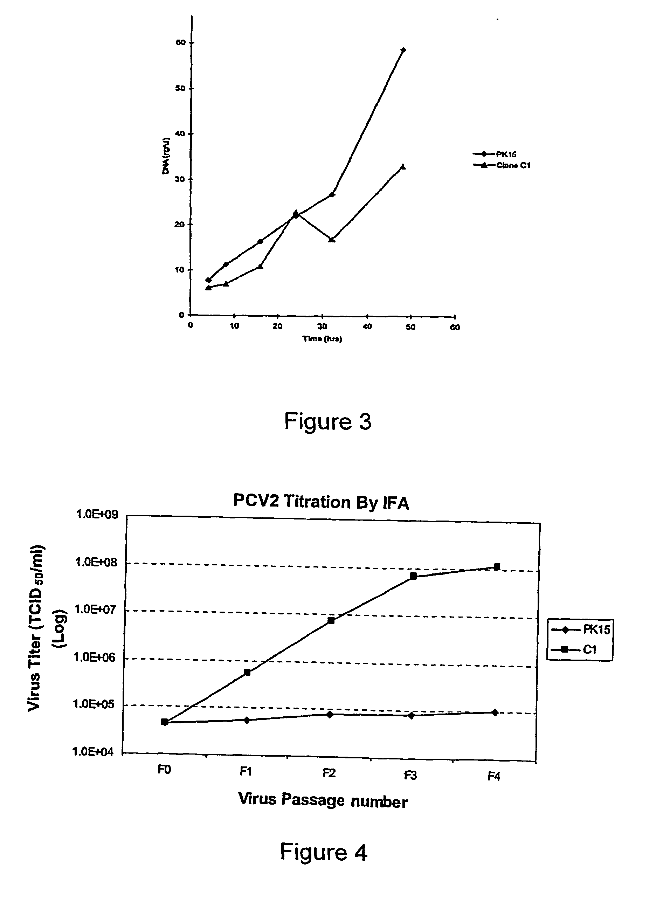

PK15 parent cells and clone C1 cells were characterized by their mean generation time in hours. Approximately 1 x 10⁵ cells were seeded into 6-well plates, and cultured in MEM/10% FBS. The cells were trypsinized, counted, and underwent DNA extraction and quantitation at 0, 4, 8, 16, 24, 32 and 48 hours post-seeding. From the cell count and DNA quantitation data, the mean generation times of PK15 parent and clone C1 cell populations were determined to be 12.1 and 14.6 hours respectively. The results shown in Figure 3 and Table 1 suggest that PK15 parent cells doubles faster than that of clone C1 cells.

Following, the titres for released virus in the culture supernatant were compared between virus yields from PK15 parent and clone C1 cells. The cells were seeded to 70% confluency in 150 cm² tissue culture flasks and infected with PCV2 (10⁴ TCID₅₀ / ml) at 6 hours post-seeding. The infected cultures were treated with D-glucosamine and maintained in culture media as previously mentioned. Finally, the virus-infected cultures were freeze-thawed three times at 4 days post infection (DPI), cells debris were pelleted at 3500 rpm at 4°C for 5 minutes and supernatant containing PCV2 virus was retrieved. PCV2 virus was serially passaged in PK15 parent and clone C1 cell lines, harvested and stored at -80°C until infectivity was determined by IFA using C1 clones. IFA results demonstrated that C1 cell clone produced a maximum virus titer of 10⁸ TCID₅₀/mL after 5 serial passages compared to a lower titer of 10⁵ TCID₅₀/mL generated from the parental PK15 cell line (Figure 4).

**Table 1**

| Cell Line | Mean Generation Time (Hours) |
|---|---|
| PK15 | 12.1 |
| Clone C1 | 14.6 |

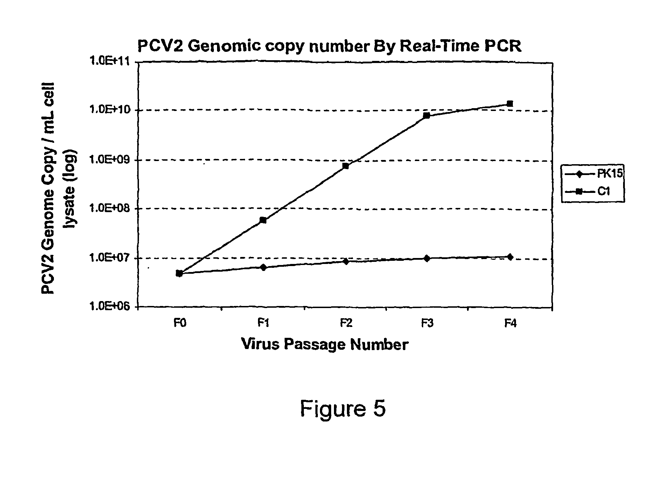

DNA replication rates of PCV2 in the parental PK15 and C1 cell clone were also assessed using a real-time PCR method. Two hundred microliters of each PCV2 infected PK15 and C1 cell lysate were harvested at 4 day post-infection (DPI) and DNA extractions were carried out using the QiaAmp DNA Mini kit (QIAGEN, Inc., Valencia, California USA). The purified DNA was then eluted in 200 microliters of sterile distill water. Real-time PCR was carried out using the Roche LightCycler system (Roche Applied Science, Indianapolis, Indiana USA). One microliter of each DNA extract was used as PCR template and a pair of PCV2 specific primers was used for the amplification (Forward primer: 5' cacctggttgtggtaaaagc 3', Reverse primer: 5' ggtctgattgctggtaatcg 3'). A PBluescript plasmid (Stratagene, La Jolla, California USA) containing PCV2 genome insert was used as standard reference. Real-time PCR quantification has shown that the genomic DNA copy number of PCV2 in 1 mL of PK15 and C1 cell lysates are 10⁷ and 10¹⁰ respectively (Figure 5).

## Claims

1. A method for the continuous production of porcine circovirus type 2 ("PCV2"), which comprises infecting a porcine kidney cell line PK15-C1 with said virus, growing said cell line under conditions suitable for cell growth; and recovering said virus produced by said cell line, wherein said porcine kidney cell line PK15-C1 is the cell line PK15-C1 deposited with the American Type Culture Collection on March 20, 2007 as PTA-8244.

2. A method for producing a substantially homogeneous cell line that is highly permissive to porcine circovirus type 2 ("PCV2") infection, which comprises (1) cultivating a heterogeneous cell population that contains cells of varying susceptibility to PCV2 infection; (2) diluting the cell culture and placing aliquots of the diluted cells into separate vessels such that each vessel contains about one cell; (3) adding PCV2 to each vessel; (4) culturing the cells and identifying a vessel that contains cells that are susceptible to PCV2 infection; and (5) culturing and maintaining a cell line from such cells, wherein said heterogeneous cell population is a PK15 cell population.

3. The method of claim 2, wherein steps 2-5 are repeated at least once.

4. The cell line PK15-C1 deposited with the American Type Culture Collection on March 20, 2007 as PTA-8244.

## Patentansprüche

1. Verfahren zur Dauerherstellung des porcinen Circovirus vom Typ 2 ("PCV2"), welches Folgendes umfasst: Infizieren einer Schweinenieren-Zelllinie PK15-C1 mit dem Virus, Züchten der Zelllinie unter für das Zellwachstum geeigneten Bedingungen und Wiedergewinnen des durch diese Zelllinie produzierten Virus, wobei die Schweinenieren-Zelllinie PK15-C1 die Zelllinie PK15-C1 ist, die am 20. März 2007 bei der American Type Culture Collection als PTA-8244 hinterlegt wurde.

2. Verfahren zur Herstellung einer im Wesentlichen homogenen Zelllinie, die höchst permissiv gegenüber einer Infektion mit dem porcinen Circovirus vom Typ 2 ("PCV2") ist, welches Folgendes umfasst: (1) Kultivieren einer heterogenen Zellpopulation, die Zellen unterschiedlicher Anfälligkeit gegen PCV2-Infektion enthält; (2) Verdünnen der Zellkultur und Platzieren von Aliquoten der verdünnten Zellen in separate Gefäße, so dass jedes Gefäß etwa eine Zelle enthält; (3) Hinzufügen von PCV2 zu jedem Gefäß; (4) Kultivieren der Zellen und Identifizieren eines Gefäßes, das Zellen enthält, die gegen PCV2-Infektion anfällig sind; und (5) Kultivieren und Aufrechterhalten einer Zelllinie aus solchen Zellen, wobei die heterogene Zellpopulation eine PK15-Zellpopulation ist.

3. Verfahren nach Anspruch 2, wobei Schritte 2 bis 5 mindestens einmal wiederholt werden.

4. Zelllinie PK15-C1, die am 20. März 2007 bei der American Type Culture Collection als PTA-8244 hinterlegt wurde.

## Revendications

1. Méthode pour la production continue du circovirus porcin de type 2 (« PCV2 »), qui comprend les étapes qui consistent à infecter une lignée cellulaire de rein porcin PK15-C1 avec ledit virus, à cultiver ladite lignée cellulaire sous des conditions appropriées à la croissance cellulaire, et à récupérer ledit virus produit par ladite lignée cellulaire, dans laquelle ladite lignée cellulaire de rein porcin PK15-C1 est la lignée cellulaire PK15-C1 ayant été déposée à la American Type Culture Collection le 20 mars 2007 sous la référence PTA-8244.

2. Méthode pour produire une lignée cellulaire substantiellement homogène qui est très permissive de l'infection par le circovirus porcin de type 2 (« PCV2 »), qui comprend les étapes qui consistent: (1) à cultiver une population de cellules hétérogènes qui contient des cellules de sensibilité variable à l'infection à PCV2 ; (2) à diluer la culture cellulaire et à placer des parties aliquotes des cellules diluées dans des récipients séparés de telle sorte que chaque récipient contienne environ une cellule ; (3) à ajouter le PCV2 dans chaque récipient ; (4) à cultiver les cellules et à identifier un récipient qui contient des cellules qui sont sensibles à l'infection à PCV2 ; et (5) à cultiver et à maintenir une lignée cellulaire à partir de telles cellules, dans laquelle la population de cellules hétérogènes est une population de cellule PK15.

3. Méthode selon la revendication 2, dans laquelle les étapes 2 à 5 sont répétées au moins une fois.

4. Lignée cellulaire PK15-C1 déposée à la American Type Culture Collection le 20 mars 2007 sous la référence PTA-8244.
